# FASCICULE DE BREVET EUROPEEN

(11) **EP 2 668 101 B1**
(45) Date de publication et mention de la délivrance du brevet: **10.06.2015**
(21) Numéro de dépôt: 12700505.6
(22) Date de dépôt: 23.01.2012
(51) Int. Cl.: B65B 3/00, A61M 5/178

(54) **DISPOSITIF STERILE A USAGE UNIQUE DE MISE EN SERINGUES DE MEDICAMENTS RADIOPHARMACEUTIQUES, SYSTEME AUTOMATIQUE ET PROCEDE DE MISE EN SERINGUES METTANT EN OEUVRE CE DISPOSITIF**
STERILE EINWEG-VORRICHTUNG ZUM BEFÜLLEN VON SPRITZEN MIT RADIOPHARMAZEUTIKA UND AUTOMATISCHES SYSTEM SOWIE VERFAHREN ZUM BEFÜLLEN VON SPRITZEN MIT DIESER VORRICHTUNG
SINGLE-USE STERILE DEVICE FOR FILLING SYRINGES WITH RADIOPHARMACEUTICAL DRUGS, AND AUTOMATIC SYSTEM AND METHOD FOR FILLING SYRINGES USING SAID DEVICE

(30) Priorité: 25.01.2011 FR 1150563
(43) Date de publication de la demande: 04.12.2013
(73) Titulaire: Commissariat à l'Énergie Atomique et aux Énergies Alternatives, 75015 Paris (FR)
(72) Inventeur: VUILLEMARD, Catherine, 91250 Saint-Germain-lès-Corbeil (FR); CHAMBELLAN, Dominique, 78000 Versailles (FR); CORPACE, Olivier, 78000 Versailles (FR); COULON, Christine, 78117 Toussus-le-Noble (FR); DUBOUE, Bruno, 91300 Massy (FR)
(74) Mandataire: Ilgart, Jean-Christophe
(86) Numéro de dépôt international: PCT/EP2012/050929
(87) Numéro de publication internationale: WO 2012/101074

(56) Documents cités:
- EP-A1- 1 860 029
- EP-A1- 2 179 926
- WO-A1-2005/002971
- WO-A1-2009/100428
- US-A- 5 911 252

## Description

### DOMAINE TECHNIQUE

L'invention concerne un dispositif stérile à usage unique de mise en seringues de médicaments radiopharmaceutiques, un système automatique et un procédé de mise en seringues mettant en oeuvre ce dispositif.

L'invention concerne plus précisément un dispositif ou « kit », stérile et à usage unique de mise en seringues d'un médicament radiopharmaceutique marqué avec des émetteurs de simples photons comme le Technétium 99m [^{99m}Tc] ou marqué avec des émetteurs de positons comme le Fluor 18 [¹⁸F], et un système automatique permettant de dispenser des seringues de médicaments radiopharmaceutiques en fonction de l'activité radiologique présente dans le corps des seringues. Ce système automatique comprend entre autre un appareil de mesure et d'identification radionucléidique du radioisotope présent dans le médicament radiopharmaceutique. L'invention concerne également un procédé de mise en seringues des médicaments radiopharmaceutiques mettant en oeuvre ce dispositif stérile ainsi que ce système automatique.

### ETAT DE LA TECHNIQUE ANTERIEURE

Le domaine de l'invention est celui de la mise en seringues de médicaments radiopharmaceutiques contenant des radioéléments artificiels appelés radionucléides. De tels médicaments sont utilisés à des fins diagnostiques ou thérapeutiques dans les services de médecine nucléaire. Ils se présentent :
- soit sous la forme de spécialités pharmaceutiques livrées prêtes à l'emploi et conditionnées en flacon,
- soit sous la forme de préparations radiopharmaceutiques fabriquées in situ et extemporanément par marquage de molécules vectrices, désignées sous le vocable de « trousse » par l'homme du métier, avec un radionucléide choisi et conditionnées en flacon.

De manière connue, la préparation de tels médicaments radiopharmaceutiques et la mise en seringues de ces préparations radiopharmaceutiques ou de ces spécialités sont réalisées dans des enceintes blindées et confinées en dépression, pourvues d'ouvertures de type ronds de gants destinées à la manipulation, dans lesquelles les opérateurs introduisent les mains. Le transfert des solutions radioactives s'effectue par le biais de seringues munies de protège-seringues blindés. Malgré l'optimisation en terme de radioprotection, l'environnement irradiant dans une enceinte blindée soumet les mains de l'opérateur à une exposition externe élevée. Les risques de contamination radioactive ou de « piqûres » de l'opérateur lors de la mise en seringues sont importants.

Différents systèmes automatisés de mise en seringues de médicaments radiopharmaceutiques, essentiellement marqués avec des émetteurs de positons comme le Fluor 18 [¹⁸F], permettent la dispensation de seringues diminuant partiellement la dosimétrie des extrémités ou « bout de doigt » des opérateurs. Mais ces systèmes automatisés n'incluent pas de système de mesure directe de l'activité radioactive des solutions. Seuls les volumes de solution sont mesurés. Le volume à prélever dans de tels systèmes est fonction de l'activité souhaitée et peut être déduit de la valeur de l'activité volumique de la solution radioactive mesurée en dehors du système automatisé. Ainsi, l'activité d'un flacon contenant le médicament radiopharmaceutique est mesurée préalablement à la mise en seringues. De même, la mesure réglementaire de l'activité de la seringue est effectuée après remplissage. Ces deux opérations de mesure conduisent à une exposition des mains de l'opérateur aux rayonnements ionisants.

D'autres systèmes automatisés de l'art connu ne comprennent pas de système de mesure intégré de l'activité radioactive des solutions. La seringue munie de son protège-seringue blindé est mesurée par le biais d'une chambre à puits indépendante du système, non incluse dans l'automate mais déjà présente dans l'enceinte blindée du service. Un système pneumatique permet le déplacement de la seringue munie de son protège-seringue de l'extérieur vers l'intérieur et de l'intérieur vers l'extérieur de la chambre à puits. La mesure de la seringue avec le protège-seringue engendre une incertitude sur la mesure de l'activité de la seringue. De même, la déconnexion de la seringue du système peut provoquer une dispersion de la solution radioactive sur le plan de travail pouvant engendrer un risque de contamination de l'opérateur.

Un système automatisé de l'art connu, décrit dans le document référencé [1] en fin de description, permet la dispensation de seringues de médicaments radiopharmaceutiques marqués avec des émetteurs de positons comme le Fluor 18 [¹⁸F]. Ce système permet de distribuer et de diluer des doses individuelles de façon aseptique tout en minimisant l'exposition des mains de l'opérateur aux rayonnements ionisants. Le risque d'exposition des mains est, malgré tout, présent lors du retrait du kit de mise en seringue utilisé, c'est-à-dire avant chaque nouvelle mise en seringues. Un détecteur de type Geiger-Müller, ou une photodiode, permet de comptabiliser en ligne la radioactivité qui est transférée dans le corps de la seringue. Cette mesure d'activité est une mesure indirecte et ne permet pas de garantir la dose délivrée. L'aiguille de la seringue est décapuchonnée et va percer un « site d'injection », ce qui peut constituer un risque de contamination biologique de l'aiguille. Il existe de même un risque de fuite de liquide radioactif sur le plan de travail, lors de la déconnexion de la seringue, ce qui constitue un risque de contamination de l'opérateur.

Le document référencé [2] décrit un système comprenant une station de remplissage conçue pour la préparation en conditions stériles de seringues de médicaments radiopharmaceutiques marqués avec des émetteurs de positons comme le Fluor 18. Ce système permet de dispenser des seringues les unes après les autres à partir d'un médicament radiopharmaceutique multi-doses. L'automate utilisé peut délivrer l'activité requise dans une seringue, grâce à une chambre de mesure et la diluer avec une solution physiologique pour le confort de l'injection. La mesure de la seringue est effectuée dans la chambre de mesure sans protège-seringue, celui-ci étant repositionné de façon automatique après la mesure. Mais ce système présente les inconvénients suivants :
- La mise en seringues d'un autre médicament nécessite le changement de l'ensemble du kit utilisé avec ouverture de la station de remplissage. Les risques de contamination radioactive ou d'irradiation de l'opérateur sont d'autant plus importants que le changement est effectué avant que l'activité résiduelle contenue dans le kit n'ait complètement décru.
- Lors de la mesure dans la chambre à puits, la seringue étant raccordée à un prolongateur, une partie de l'activité contenue dans celui-ci engendre systématiquement une erreur sur la mesure de l'activité contenue dans la seringue.

L'invention a pour objet de résoudre ces différents problèmes techniques en proposant un dispositif stérile se présentant sous la forme d'un « kit de mise en seringues » conditionné sous enveloppe stérile de type « blister » apte à être placé sur un système automatique de mise en seringues. Ce système est destiné à être introduit à l'intérieur d'une enceinte blindée et confinée préexistante d'une installation de médecine nucléaire et permettre la dispensation séquentielle de seringues (dénommées « seringues patients ») contenant des médicaments radiopharmaceutiques marqués avec des émetteurs de simples photons ou marqués avec des émetteurs de positons, avec une activité radioactive bien définie, cette activité étant mesurée par le biais d'un détecteur qui est susceptible de fournir également une analyse spectrale et ainsi d'identifier le radionucléide présent dans le médicament radiopharmaceutique.

L'invention a également pour objet de dispenser des seringues contenant des médicaments radiopharmaceutiques de nature différente en fonction de la demande et ceci sans avoir à changer le dispositif entre deux dispensations.

### EXPOSÉ DE L'INVENTION

L'invention concerne un dispositif stérile à usage unique de mise en seringues d'un médicament radiopharmaceutique, qui comprend au moins deux prolongateurs raccordés chacun, en une première extrémité, à une aiguille spinale apte à percuter un flacon, contenant un médicament radiopharmaceutique, positionné dans des moyens de confinement des rayonnements ionisants et, en une seconde extrémité, à un premier connecteur, caractérisé en ce que ce premier connecteur est un connecteur de sécurité apte à être connecté à un second connecteur de sécurtié solidaire d'une seringue patient pour permettre le remplissage de cette seringue patient, empêcher toute contamination par écoulement et assurer une sécurité microbiologique lors de leur déconnexion, le premier connecteur de sécurité étant en position fermée tant qu'il n'est pas relié au second connecteur de sécurtié, la connexion de ce premier et de ce second connecteurs de sécurité permettant une circulation de fluide, et en ce que le dispositif comprend en outre une console sur laquelle sont fixés au moins deux premiers connecteurs de sécurité et qui est disposée sur un axe linéaire horizontal pouvant être animé d'un mouvement de translation horizontal.

Dans l'invention ainsi revendiquée la console n'est pas munie, comme dans le document référencé [3], de vannes permettant de sélectionner le flux à faire circuler dans une rampe. La console de l'invention sert de support à au moins deux connecteurs de sécurité reliés à autant de prolongateurs, ces premiers connecteurs de sécurité étant en position fermée (d'où le nom de connecteur « de sécurité ») tant qu'ils ne sont pas reliés à un second connecteur de sécurité, solidaire d'une seringue patient. La connexion d'un premier et d'un second connecteurs de sécurité, ouvre la circulation de fluide sur la voie choisie : le choix de la voie étant effectué par déplacement de la console le long d'un axe linéaire horizontal permettant d'amener le premier connecteur de sécurité sélectionné en vis-à-vis du second connecteur de sécurité. La rampe du document référencé [3] munie de ses vannes peut être assimilée à une console de sélection. Mais dans l'invention, la console est une console de support de connecteurs, apte à être placée sur un axe horizontal pouvant être animé d'un mouvement de translation horizontal. Par ailleurs, dans l'invention c'est la connexion des premier et second connecteurs de sécurité qui fait fonction de vanne. Lorsqu'ils ne sont pas connectés, ils sont fermés et permettent que, tant du côté des flacons que du côté seringue patient, les solutions radioactives ne s'écoulent pas (les prolongateurs sont toujours remplis). La connexion d'un premier connecteur de sécurité positionné en face d'un second connecteur de sécurité ouvre la connexion et permet l'écoulement de la solution vers la seringue patient. Le mot « sécurité » est donc important.

L'invention concerne également un système automatique de mise en seringues d'au moins un médicament radiopharmaceutique, par exemple marqué avec des émetteurs de simples photons ou marqués avec des émetteurs de positons, conditionné en flacon, mettant en oeuvre un dispositif tel que défini ci-dessus, comprenant :
- au moins deux pompes, par exemple péristaltiques, susceptibles de recevoir chacune au moins un prolongateur du dispositif de mise en seringues,
- des moyens de confinement des rayonnements ionisants d'au moins deux flacons contenant chacun un médicament radiopharmaceutique,
- au moins un appareil de mesure de l'activité d'une seringue patient confinée dans un protège-seringue, permettant l'identification radionucléidique du radioélément ou l'identification de la pureté radionucléidique d'un médicament radiopharmaceutique, cet appareil de mesure étant confiné dans une protection blindée,

- un absorbant positionné entre la seringue patient et l'appareil de mesure permettant d'homogénéiser et d'uniformiser la réponse de l'appareil de mesure,
- un axe linéaire vertical muni de moyens de commande automatisée, susceptible de recevoir la seringue patient munie de son protège-seringue et d'un second connecteur de sécurité, cet axe linéaire pouvant être animé d'un mouvement de translation vertical descendant et ascendant réalisant ainsi la connexion ou la déconnexion d'un des au moins deux premiers connecteurs de l'équerre de sélection avec le second connecteur de la seringue patient.
- un axe linéaire horizontal muni de moyens de commande automatisée susceptible de recevoir la console, cet axe linéaire pouvant être animé d'un mouvement de translation horizontal permettant de positionner un des au moins deux premiers connecteurs de sécurité de la console, face au second connecteur de sécurité positionné sur la seringue patient,
- un système informatique, dans lequel est implanté un logiciel de mise en seringue, apte à piloter ces différents moyens de commande.

Les moyens de confinement peuvent être fixés sur un support garantissant le chauffage et/ou l'agitation des flacons.

Le dispositif stérile de mise en seringues d'au moins un médicament radiopharmaceutique une fois positionné dans ce système automatique est apte à recevoir de façon séquentielle des seringues que l'on souhaite dispenser avec des activités précises (dénommées « seringues patient »). Chaque seringue patient est munie d'un protège-seringue blindé ainsi que d'un second connecteur de sécurité.

Pendant la phase de remplissage de chaque seringue patient, un second connecteur de sécurité placé sur le corps de chaque seringue patient vient se fixer sur un premier connecteur de la console de sélection. Ces premier et second connecteurs de sécurité ainsi montés en série permettent le remplissage d'une seringue patient, empêchent toute contamination par écoulement et assurent une sécurité microbiologique lors de la déconnexion.

Le système automatique permet de recevoir, au niveau de la console, la seringue patient munie de son protège-seringue. Pour un médicament radiopharmaceutique marqué avec des émetteurs de simples photons, ce protège-seringue possède une fenêtre amovible assurant ainsi la mesure de l'activité contenue dans la seringue. Pour un médicament radiopharmaceutique marqué avec des émetteurs de positons, le protège-seringue est dépourvu de fenêtre amovible et la mesure de l'activité de la seringue se fait au travers de la protection.

Ce système automatique permet avantageusement d'effectuer une mesure directe de l'activité des seringues patient que l'on souhaite dispenser sans soumettre les mains de l'opérateur aux rayonnements ionisants. Le corps de chaque seringue patient se remplit de son contenu de façon passive. Le remplissage s'interrompt lorsque l'activité demandée est obtenue. On limite ainsi l'intervention de l'opérateur. On limite l'irradiation « bout de doigts » et en même temps les risques d'accidents liés aux manipulations de substances radioactives et les risques de piqûres.

L'invention concerne également un procédé in situ de mise en seringues d'au moins un médicament radiopharmaceutique conditionné en flacon, par exemple marqué avec des émetteurs de simples photons ou marqué avec des émetteurs de positons, conditionné en flacon, comprenant les étapes automatiques suivantes :
- une première étape de positionnement du dispositif stérile de l'invention sur le système automatique de mise en seringues et plus particulièrement de positionnement des au moins deux prolongateurs sur les au moins deux pompes, par exemple péristaltiques et de raccordement de ces prolongateurs aux flacons contenant chacun un médicament radiopharmaceutique ; ces flacons étant préalablement positionnés dans des moyens de confinement des rayonnements ionisants et comprenant éventuellement un support permettant le chauffage et/ou l'agitation,
- une seconde étape de positionnement de la seringue patient munie de son protège-seringue blindé ainsi que du second connecteur de sécurité sur l'axe linéaire vertical en position la plus haute,
- une troisième étape de sélection des paramètres dans un logiciel de mise en seringue et de lancement du logiciel,
- une quatrième étape de positionnement automatique de la console par le biais de la commande linéaire horizontale, cette quatrième étape étant aussi une étape de positionnement automatique de la seringue patient (en position basse) face à l'appareil de mesure et de connexion d'un premier et d'un second connecteurs de sécurité par le biais de la commande verticale automatisée, le mouvement vertical descendant engendrant non seulement la connexion des premier et second connecteurs de sécurité mais aussi l'ouverture éventuelle de la fenêtre amovible du protège seringue dans le cadre de la mise en seringue d'un médicament radiopharmaceutique marqué avec des émetteurs de simples photons,
- une cinquième étape de remplissage partiel par le biais d'au moins un prolongateur d'au moins une seringue patient en fonction du médicament radiopharmaceutique et de l'activité radioactive souhaités, l'activité radioactive ainsi que l'identification radionucléidique du radioélément composant le médicament radiopharmaceutique étant déterminées par l'appareil de mesure,
- une sixième étape de déconnexion des connecteurs de sécurité montés en série et de remontée de la seringue patient le long de l'axe linéaire vertical par le biais de la commande automatisée, la remontée de cette seringue patient engendrant la fermeture si besoin d'une fenêtre amovible du protège-seringue. Cette sixième étape peut être aussi une étape de dilution de la solution radioactive avec une solution saline, pour les médicaments radiopharmaceutiques marqués avec des positons. En effet, pour de tels médicaments, une dilution peut être nécessaire après la sixième étape. La seringue patient peut alors être connectée de nouveau à la console pour une septième étape de dilution avec une solution saline.

A la fin du procédé de l'invention, la mise en seringues du médicament radiopharmaceutique est terminée. L'opérateur peut prendre la seringue patient munie de son protège seringue en vue de son utilisation sans risque de contamination ou d'irradiation et relancer la procédure d'une nouvelle mise en seringues à partir de la seconde étape.

Le procédé de l'invention est donc automatisé depuis le positionnement du dispositif stérile à usage unique sur le système de mise en seringues jusqu'à l'obtention finale des seringues patient remplies de solutions radioactives.

Les mains de l'opérateur ne sont, à aucun moment, exposées aux rayonnements ionisants générés par le radionucléide, ce qui permet de diminuer considérablement la dosimétrie « bout de doigt » des préparateurs et des manipulateurs en électroradiologie et les risques de piqûres. Les risques de mauvaises manipulations et de piqûres sont donc très limités.

### BRÈVE DESCRIPTION DES DESSINS

La figure 1 est une vue schématique du dispositif stérile à usage unique de l'invention permettant la mise en seringues de médicaments radiopharmaceutiques marqués avec des émetteurs de simples photons ou marqués avec des émetteurs de positons.
La figure 2 est une vue schématique générale du système automatique de mise en seringues selon l'invention, dans lequel est intégré le dispositif de mise en seringues illustré sur la figure 1 pour au moins un médicament radiopharmaceutique marqué avec des émetteurs de simples photons ou avec des émetteurs de positons.
La figure 3 est une représentation schématique de la commande automatisée d'un axe linéaire vertical qui permet de positionner une seringue munie de son protège seringue et d'un second connecteur sur un premier connecteur d'une console.

### EXPOSÉ DÉTAILLÉ DE MODES DE RÉALISATION PARTICULIERS

L'invention concerne un dispositif, ou kit, stérile de mise en seringues d'au moins un médicament radiopharmaceutique, par exemple marqué avec des émetteurs de simples photons comme le Technétium 99 [^{99m}Tc] ou marqué avec des émetteurs de positons comme le Fluor 18 [¹⁸F], apte à être positionné sur un système automatique de mise en seringues.

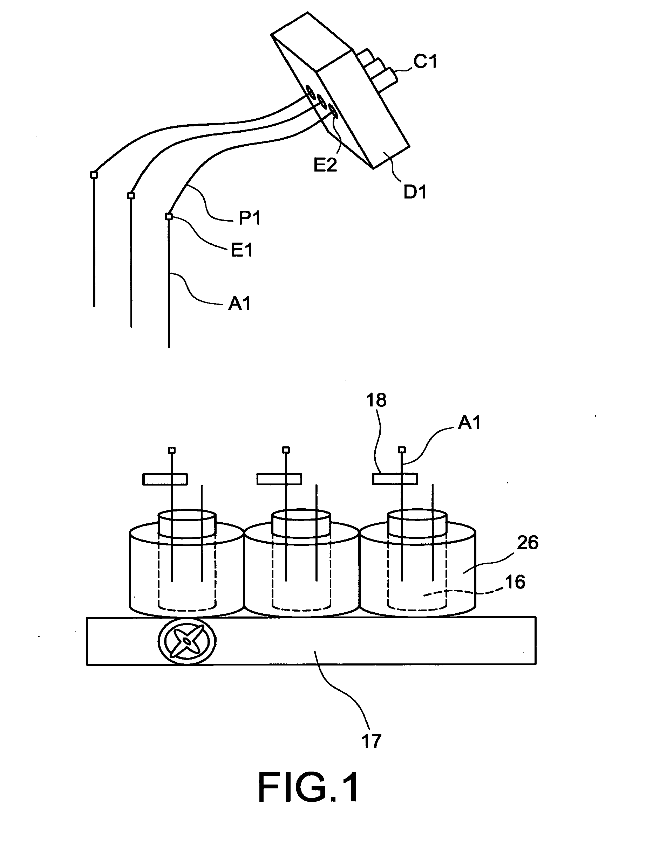

Comme illustré sur la figure 1, un tel dispositif comporte :
- au moins deux prolongateurs P1 raccordés chacun, en une première extrémité E1, à une aiguille spinale A1, et en une seconde extrémité E2, à un premier connecteur de sécurité C1 apte à être connecté à un second connecteur de sécurité C2 solidaire d'une seringue patient,
- une console D1, qui peut être une équerre parallépipédique, sur laquelle sont intégrés en partie et de façon perpendiculaire au moins deux premiers connecteurs C1 disposés de façon espacée, cette console D1 pouvant être placée sur un axe linéaire horizontal, qui est animé d'un mouvement de translation, permettant de positionner un des au moins deux premiers connecteurs C1 de la console de sélection D1, face à un second connecteur C2 positionné sur la seringue patient S1.

Les aiguilles spinales A1 permettent de percuter des flacons 26 contenant chacun un médicament radiopharmaceutique, positionnés dans des moyens de confinement des rayonnements ionisants. Les médicaments radiopharmaceutiques sont transférés par le biais des prolongateurs P1 pour une dispensation partielle et séquentielle dans des seringues patients S1,

Seuls les embouts des premiers connecteurs C1 ressortent de la console D1 permettant ainsi la connexion de chaque connecteur C1 avec un second connecteur C2 d'une seringue patient S1. Un premier connecteur C1 est susceptible d'être raccordé à un second connecteur C2, pendant une phase de remplissage de ladite seringue, par le biais de commandes automatisées.

Ainsi, un médicament radiopharmaceutique peut être transféré partiellement et séquentiellement dans des seringues patients S1. Lors de la déconnexion d'un premier connecteur C1 et d'un second connecteur C2, ces connecteurs de sécurité empêchent la contamination par écoulement et assurent la sécurité microbiologique des solutions radioactives se trouvant en amont et en aval de ces connecteurs.

La seringue patient S1 contenant un médicament radiopharmaceutique, associée à son second connecteur C2, est ainsi prête à l'emploi. L'opérateur peut la prendre en vue de son utilisation.

Le dispositif stérile de l'invention permet de recevoir une seringue patient S1, elle même susceptible d'accueillir un volume déterminé de médicament radiopharmaceutique avec une activité radioactive précise. Dans le cas de médicaments radiopharmaceutiques marqués avec des positons, si une dilution s'avère nécessaire, une solution de dilution est placée dans un des flacons à la place d'un médicament radiopharmaceutique et est transférée partiellement dans la seringue patient S1 pour diluer le médicament radiopharmaceutique. Une telle dilution nécessite la déconnexion des deux connecteurs de sécurité C1 et C2. La console D1 positionnée sur l'axe linéaire horizontal subit alors un mouvement de translation horizontal permettant de placer le premier connecteur C1 du prolongateur contenant la solution de dilution, face à la seringue patient S1 munie de son second connecteur C2. Ces deux connecteurs de sécurité C1 et C2 sont de nouveau connectés par le biais de la commande automatisée. La dilution est ainsi réalisée.

Dans un exemple de réalisation avantageux, le dispositif de mise en seringues de l'invention est réalisé à partir de matériaux transparents compatibles avec la (ou les) solution(s) radioactive(s) utilisée(s). Ces matériaux peuvent être en polycarbonate, ou en polyéthylène. Les prolongateurs P1 peuvent être réalisés en polyéthylène, avec un diamètre interne de 1mm et une longueur de 15cm. L'aiguille spinale A1 peut être une aiguille de type Becton Dickinson (20GA 3,50 in 0,9x90mm). Les premier et second connecteurs de sécurité C1 et C2 peuvent être respectivement à embout mâle et à embout femelle et être réalisés en polycarbonate et silicone. Ces connecteurs de sécurité C1 et C2 peuvent être connectables entre eux de façon automatique par enfoncement du second connecteur C2 dans le premier connecteur C1, la déconnexion de ces deux connecteurs C1 et C2 étant aussi automatique, de manière à empêcher toute contamination par écoulement et assurer la sécurité microbiologique des solutions radioactives, tout en évitant tout risque de piqûres. Les seringues patient S1 peuvent être des seringues de type « BD Plastipak » (Société Becton Dickinson SA, Espagne) en polypropylène de capacité d'au moins 2,5ml pour des médicaments radiopharmaceutiques émetteurs de simples photons et d'au moins 10ml pour des médicaments radiopharmaceutiques émetteurs de positons. La console D1 peut être une équerre parallépipédique en polycarbonate ou en polyéthylène. La solution de dilution utilisée peut être par exemple une solution isotonique injectable comme par exemple une solution de Chlorure de Sodium 0,9%.

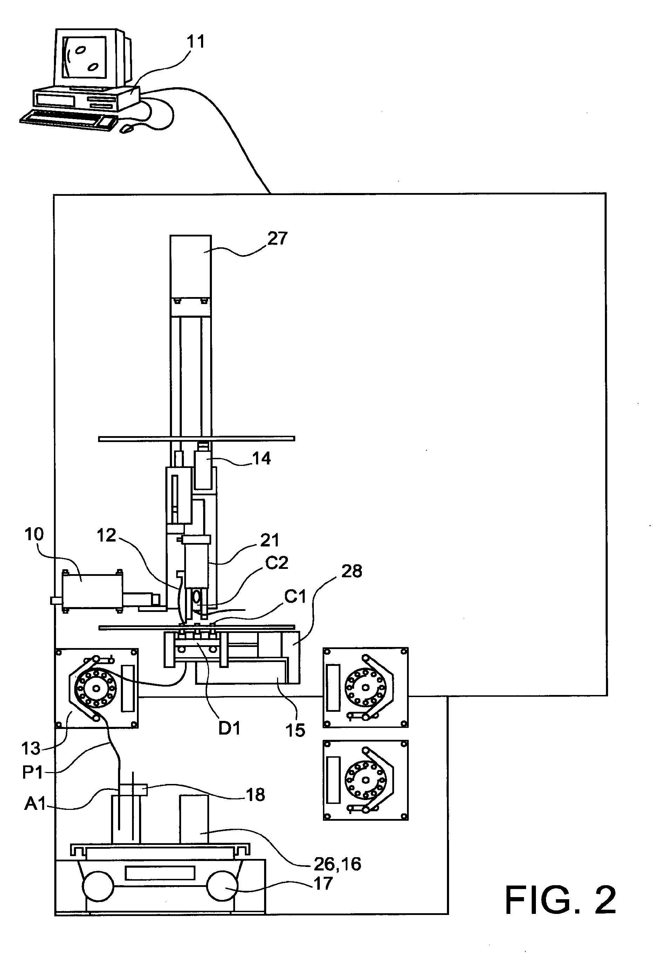

Le dispositif stérile de l'invention, ou kit à usage unique, peut être intégré dans un système automatique de mise en seringue schématisé en figure 2, permettant la préparation de plusieurs seringues patient S1 dispensées les unes après les autres. Ces seringues patient S1 peuvent renfermer des médicaments radiopharmaceutiques émetteurs de simples photons, ou des médicaments radiopharmaceutiques émetteurs de positons.

Ce système automatique comprend un appareil 10 de mesure automatique sans intervention humaine de l'activité radiologique présente dans les seringues patients S1. Cet appareil de mesure 10, calibré pour les radionucléides suivants : le Technétium 99 [^{99m}Tc] et le Fluor 18 [¹⁸F], est confiné dans une protection blindée permettant d'être isolé de l'ambiance radioactive environnante. Cet appareil de mesure 10 est relié à un système informatique 11, dans lequel est implanté un logiciel de mise en seringue.

Cet appareil de mesure 10 est fixe et se situe par exemple à une distance de 10 cm de la seringue patient S1 à mesurer. Cet appareil de mesure 10 comprend par exemple un détecteur semi-conducteur de type CdTe associé à une chaîne d'acquisition miniaturisée permettant une mesure spectrométrique, par exemple un spectromètre gamma. Ce spectromètre gamma permet ainsi de réaliser une identification radionucléidique du radioélément (identification de la pureté radionucléidique de la solution radioactive). Cet appareil de mesure 10 permet d'acquérir le spectre de photons émis par la solution contenue dans la seringue patient S1 de façon non ambiguë. Il présente donc une résolution en énergie suffisante, par exemple 10% à 140 keV y compris à des flux de photons élevés, par exemple quelques dizaines de milliers de coups par seconde.

Un absorbant 12 peut être positionné entre la seringue patient S1 que l'on souhaite dispenser et l'appareil de mesure 10. Cet absorbant 12 permet d'homogénéiser et d'uniformiser la réponse de l'appareil de mesure sur la hauteur de la seringue patient S1 pour compenser les variations de distance entre tout point de la seringue patient S1 et le détecteur. Cet absorbant 12 permet de mesurer l'activité indépendamment du volume de liquide dans la seringue S1. Cet absorbant 12 peut être constitué d'inox, d'aluminium, de tungstène ou autre. Cet absorbant 12 peut également être constitué d'une combinaison de différents matériaux.

Cet appareil de mesure 10 calibré pour le radionucléide choisi et pour le conditionnement des seringues patients S1 permet de mesurer l'activité radiologique qui est transférée passivement dans le corps de la seringue patient S1. A cette mesure d'activité radioactive s'ajoute une mesure du spectre gamma du radionucléide permettant une identification radionucléidique du médicament radiopharmaceutique présent dans la seringue patient S1.

La solution radioactive dispensée passivement par le biais d'une d'au moins deux pompes 13, par exemple péristaltique, dans la seringue patient S1 est issue d'un des au moins deux flacons contenant chacun un médicament radiopharmaceutique. Ces pompes 13 sont destinées à commander le remplissage et/ou l'extraction d'un volume partiel de solution radioactive de ladite seringue S1. Ces pompes 13 sont raccordées au système informatique 11.

Le système automatique comporte aussi un moyen de commande 14 de l'axe linéaire vertical 27 susceptible de recevoir la seringue patient S1 ainsi qu'un moyen de commande 15 de l'axe linéaire horizontal 28 susceptible de recevoir la console de sélection D1 du kit.

Le moyen de commande 14 est un moteur générant un mouvement de translation vertical descendant ou ascendant de la seringue patient S1 réalisant ainsi son positionnement face à l'appareil de détection 10 ou son éloignement, la connexion ou la déconnexion des connecteurs de sécurité et éventuellement l'ouverture ou la fermeture de la fenêtre amovible du protège seringue dans le cadre de la mise en seringue d'un médicament radiopharmaceutique marqué avec des émetteurs de simples photons. Le moyen de commande 15 est un moteur générant un mouvement de translation horizontal (droite à gauche ou gauche à droite) de l'axe linéaire horizontal permettant de positionner un des au moins deux premiers connecteurs C1 de la console, face à un second connecteur C2 positionné sur la seringue patient S1. Ces moyens de commande 14 et 15 sont également raccordés au système informatique 11.

Comme illustré sur la figure 2, le système automatique comporte aussi au moins deux flacons 16 pourvus de moyens 26 de confinement des rayonnements ionisants tels qu'une protection en tungstène ou en plomb fixés sur un support 17 qui peut garantir le chauffage et/ou l'agitation des flacons 16 contenant chacun une préparation radiopharmaceutique marquée avec des émetteurs de simples photons.

Certaines préparations radiopharmaceutiques émetteurs de simples photons demandent un chauffage final à 100°C pendant un temps bien défini, suivi d'une agitation ou seulement d'une simple agitation préalablement à leur transfert séquentiel dans les seringues patients S1. Le chauffage peut alors nécessiter la présence d'un filtre 0,22µm 18 fixé sur une aiguille 19 surmontant chaque flacon 16 contenant chacun une préparation radiopharmaceutique différente.

Pour des préparations radiopharmaceutiques émetteurs de positons, ces flacons 16 pourvus de moyens de confinement des rayonnements ionisants peuvent être de simples pots blindés en tungstène ou en plomb fixés sur un simple support 17 n'assurant ni le chauffage ni l'agitation.

Le chauffage des flacons peut s'effectuer par conduction de chaleur à partir d'éléments chauffants du type résistances chauffantes. L'agitation de ces flacons peut se faire par mise en vibration ou en translation du support accueillant les flacons.

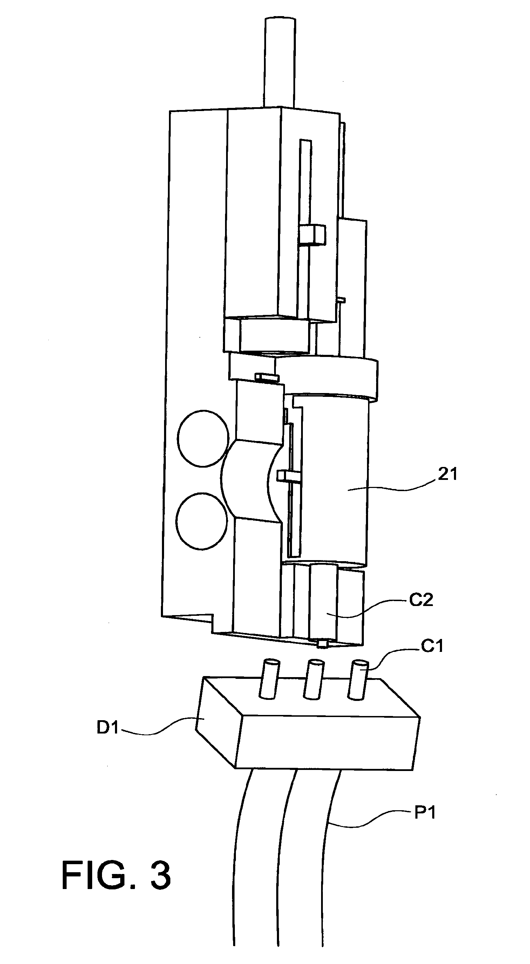

Comme illustré sur la figure 3, les commandes automatisées 14 et 15 nécessitent :
- un positionnement manuel par l'opérateur de la seringue patient S1 munie de son protège-seringue 21 et de son connecteur de sécurité C2 sur l'axe linéaire vertical par enclenchement de l'ensemble dans un logement prévu a cet effet situé sur l'axe linéaire,
- un mouvement de translation horizontal de l'axe linéaire permettant le positionnement d'un des au moins deux premiers connecteurs C1 de l'équerre D1, face au second connecteur C2 positionné sur la seringue patient S1,
- un mouvement de translation vertical descendant de la seringue patient S1 réalisant ainsi son positionnement face à l'appareil de détection 10 ainsi que la connexion du second connecteur C2 de la seringue patient S1 avec un des au moins deux premiers connecteurs C1 de l'équerre D1. Ce mouvement vertical descendant engendre aussi l'ouverture de la fenêtre amovible du protège seringue dans le cadre de la mise en seringue d'un médicament radiopharmaceutique marqué avec des émetteurs de simples photons. L'ouverture de cette fenêtre peut être provoquée par butée de celle-ci sur un élément fixe par rapport au mouvement de translation vertical.

Le remplissage de la seringue patient S1 peut ainsi s'effectuer passivement, la seringue S1 ainsi remplie étant alors prête à l'emploi.

La déconnexion d'un premier connecteur C1 et d'un second connecteur C2 se fait par le biais de la commande automatisée 14 Un mouvement vertical ascendant de la seringue patient S1 garantit non seulement l'éloignement de la seringue patient S1 mais aussi la fermeture de la fenêtre amovible du protège seringue 21.

Le second connecteur de sécurité C2 reste positionné sur la seringue patient S1, ce qui permet de garantir ainsi l'absence de contamination par écoulement du liquide radioactif et le maintien de la sécurité microbiologique de la solution radioactive.

Le protège-seringue 21, par exemple en tungstène, positionné sur le corps de la seringue patient S1 permet de protéger les mains de l'opérateur contre l'irradiation des rayonnements.

Pour les médicaments radiopharmaceutiques marqués avec des émetteurs de simples photons, les protège-seringues 21 par exemple en tungstène positionnés sur les seringues patients S1 présentent une fenêtre amovible pour permettre la mesure de l'activité. Pour les médicaments radiopharmaceutiques marqués avec des émetteurs de positons, les protège-seringues n'en possèdent pas.

La présente invention concerne également un procédé de mise en seringue d'au moins un médicament radiopharmaceutique mettant en oeuvre le dispositif de l'invention et le système automatique de l'invention. Ce procédé de mise en seringues, après positionnement manuel d'un protège-seringue sur chaque seringue patient S1 munie de son second connecteur de sécurité C2 par glissement du corps d'une seringue S1 dans un protège-seringue, comprend les étapes suivantes :
1. une première étape de mise en place manuelle par l'opérateur du dispositif stérile de l'invention sur le système automatique de mise en seringues de l'invention, qui comprend :
   - une étape de positionnement de la console D1 sur l'axe linéaire horizontal 28,
   - une étape de positionnement d'au moins deux prolongateurs P1 sur les au moins deux pompes 13,
   - une étape de raccordement de ces prolongateurs P1 à des flacons 16 contenant chacun un médicament radiopharmaceutique, ces flacons 16 étant préalablement positionnés dans les moyens 26 de confinement des rayonnements ionisants et éventuellement sur un support 17 permettant le chauffage et / ou l'agitation de ces flacons 16,
2. une seconde étape de positionnement manuelle par l'opérateur d'une première seringue patient S1 munie de son protège-seringue blindé 21 ainsi que du second connecteur de sécurité C2 sur l'axe linéaire vertical 27,
3. une troisième étape de sélection des paramètres de dispensation dans le logiciel de mise en seringues du système informatique 11, en choisissant le médicament radiopharmaceutique à dispenser ainsi que l'activité à transférer et éventuellement pour les médicaments radiopharmaceutiques émetteurs de positons le volume de dilution à rajouter, et de lancement de ce logiciel par validation du positionnement effectif du dispositif de l'invention et de la première seringue patient S1 ainsi que les paramètres de dispensation,
4. une quatrième étape de positionnement automatique de la console D1 par le biais de la commande linéaire horizontale, cette étape 4 étant aussi une étape de positionnement automatique par le biais de la commande verticale de la seringue patient S1 face à l'appareil de mesure 10 et de connexion des premier et second connecteurs de sécurité C1 et C2, le mouvement vertical descendant engendrant non seulement la connexion de ces connecteurs de sécurité mais aussi l'ouverture éventuelle de la fenêtre amovible d'un protège seringue 21 dans le cadre de la mise en seringues d'un médicament radiopharmaceutique marqué avec des émetteurs de simples photons,
5. une cinquième étape de remplissage partiel par le biais d'un prolongateur P1 d'une seringue patient S1 en fonction de l'activité souhaitée, qui est mesurée par l'appareil de mesure 10, le remplissage dans le corps de la seringue patient S1 se faisant passivement,
6. une sixième étape de déconnexion du premier et du second connecteurs de sécurité C1 et C2, par le biais de la commande automatisée 14, celle-ci génèrant aussi la remontée de la seringue patient S1 le long de l'axe linéaire vertical 27 qui engendre la fermeture si besoin de la fenêtre amovible du protège-seringue 21,
7. une éventuelle septième étape de dilution de la solution radioactive avec une solution saline parfois nécessaire pour les médicaments radiopharmaceutiques marqués avec des positons, la seringue patient S1 pouvant alors être connectée de nouveau à la console D1.

Pour les médicaments radiopharmaceutiques émetteurs de simples photons, la mesure des rayonnements d'énergie 140 keV pour le Technétium 99 [^{99m}Tc] par l'appareil de mesure 10, nécessite l'ouverture de la fenêtre amovible du protège seringue 21 pour permettre une lecture directe sans atténuation.

Dans la cinquième étape, le transfert partiel de préparations radiopharmaceutiques dans les prolongateurs et les seringues patient S1 n'est effectué qu'après finalisation des préparations radiopharmaceutiques émetteurs de simples photons c'est-à-dire soit après un chauffage final à 100°C pendant un temps bien défini suivi d'une agitation ou seulement après une simple agitation, le chauffage et /ou l'agitation étant réalisés par le support 17 des flacons 16. Le chauffage et ou l'agitation sont donc des étapes préalables au transfert partiel dans les prolongateurs P1 et les seringues patient S1.

Le procédé de l'invention s'arrête lorsque l'activité demandée est obtenue, un écart entre l'activité souhaitée et l'activité obtenue sur la mesure de la seringue patient ne devant pas excéder 5 %. L'activité radioactive ainsi que l'identification radionucléidique du radioélément présent dans le médicament radiopharmaceutique sont déterminées par le spectromètre gamma que constitue l'appareil de mesure 10. Cette identification radionucléidique constitue l'un des contrôles qualité obligatoire à effectuer avant la libération de la préparation radiopharmaceutique. L'activité radioactive présente dans la seringue patient S1 est enregistrée et validée dans le logiciel informatique.

A la fin du procédé de l'invention, le second connecteur de sécurité C2 reste positionné sur la seringue patient S1, ce qui garantit l'absence de contamination par écoulement du liquide radioactif et le maintien de la sécurité microbiologique de la solution radioactive. La commande automatisée 14 de l'axe linéaire vertical 27 garantit non seulement la déconnexion des connecteurs de sécurité C1 et C2 mais aussi le mouvement vertical ascendant de la seringue patient S1, favorisant ainsi son éloignement par rapport à la console D1. Lorsqu'une dilution s'avère nécessaire et ceci afin d'avoir un volume suffisant à injecter, le second connecteur de sécurité C2 de la seringue patient S1 est de nouveau connecté au premier connecteur de sécurité C1 du prolongateur P1 contenant la solution de dilution par le biais de la commande automatisée 14, après que la console D1 ait effectué un mouvement linéaire horizontal via la commande automatisée 15. Après dilution, la commande automatisée 14 de l'axe linéaire vertical 27 engendre la déconnexion des connecteurs de sécurité donc la libération de la seringue patient S1 et sa remontée le long de l'axe linéaire vertical 27.

La mise en seringues du médicament radiopharmaceutique est alors terminée. La seringue patient S1 est ainsi prête à l'emploi. L'opérateur peut prendre la seringue patient S1 en vue de son utilisation sans risque de contamination ou d'irradiation.

Les informations relatives à la seringue du patient par exemple la date de mise en seringue, l'identité du patient, l'activité radioactive présente dans la seringue patient S1 sont enregistrées dans le logiciel du système informatique 11. L'injection du médicament radiopharmaceutique à l'aide de la seringue patient S1 s'effectue en présence du connecteur C2.

Une nouvelle mise en seringues peut avoir lieu sans avoir à changer le dispositif de l'invention positionné sur le système automatique de l'invention en commençant le procédé à partir de la seconde étape.

### REFERENCES

[1] WO 2005/002971
[2] EP 1 860 029
[3] EP 2 179 926

## Revendications

1. Dispositif stérile à usage unique de mise en seringues d'un médicament radiopharmaceutique, qui comprend au moins deux prolongateurs (P1), raccordés chacun, en une première extrémité (E1), à une aiguille spinale (A1) apte à percuter un flacon (16), contenant un médicament radiopharmaceutique, positionné dans des moyens de confinement des rayonnements ionisants et, en une seconde extrémité (E2), à un premier connecteur,
**caractérisé en ce que** ce premier connecteur est un connecteur de sécurité (C1) apte à être connecté à un second connecteur de sécurité (C2) solidaire d'une seringue patient (S1) pour permettre le remplissage de cette seringue patient, empêcher toute contamination par écoulement et assurer une sécurité microbiologique lors de leur déconnexion, le premier connecteur de sécurité étant en position fermée tant qu'il n'est pas relié au second connecteur de sécurité, la connexion de ce premier et de ce second connecteurs de sécurité permettant une circulation de fluide,
- et **en ce que** le dispositif comprend en outre une console (D1) sur laquelle sont fixés au moins deux premiers connecteurs de sécurité (C1) et qui est disposée sur un axe linéaire horizontal pouvant être animé d'un mouvement de translation horizontal.

2. Dispositif selon la revendication 1, dans lequel le médicament radiopharmaceutique est marqué avec des émetteurs de simples photons.

3. Dispositif selon la revendication 1, dans lequel le médicament radiopharmaceutique est marqué avec des émetteurs de positons.

4. Dispositif selon la revendication 1, dans lequel les premier et second connecteurs de sécurité (C1, C2) sont aptes à être montés en série.

5. Système automatique de mise en seringues d'au moins un médicament radiopharmaceutique, conditionné en flacon mettant en oeuvre un dispositif selon l'une quelconque des revendications précédentes, comprenant :
- au moins deux pompes (13), susceptibles de recevoir chacune au moins un prolongateur (P1),
- des moyens de confinement des rayonnements ionisants, d'au moins deux flacons contenant chacun un médicament radiopharmaceutique,
- au moins un appareil de mesure (10) de l'activité d'une seringue patient (S1) confinée dans un protège-seringue permettant l'identification radionucléidique du radioélément ou l'identification de la pureté radionucléique d'un médicament radiopharmaceutique, cet appareil de mesure (10) étant confiné dans une protection blindée,
- un absorbant (12) positionné entre la seringue patient et l'appareil de mesure (10) permettant d'homogénéiser et d'uniformiser la réponse de l'appareil de mesure,
- un axe linéaire vertical (27) muni de moyens de commande automatisée, susceptible de recevoir la seringue patient munie de son protège-seringue et d'un second connecteur de sécurité (C2),
- un axe linéaire horizontal (28) muni de moyens de commande automatisée susceptible de recevoir la console de sélection (D1),
- un système informatique (11), dans lequel est implanté un logiciel de mise en seringues, apte à piloter ces différents moyens de commande.

6. Système selon la revendication 5, dans lequel les moyens de confinement (26) des rayonnements ionisants sont des protections en tungstène ou en plomb fixés sur un support (17).

7. Système selon la revendication 6, dans lequel, dans le cas d'un médicament radiopharmaceutique marqué avec des émetteurs simples photons, le support (17) assure également le chauffage et/ou l'agitation des flacons.

8. Système selon la revendication 5, comprenant au moins un absorbant (12) positionné entre la seringue patient (S1) et l'appareil de mesure (10).

9. Procédé de mise en seringues d'au moins un médicament radiopharmaceutique conditionné en flacon, **caractérisé en ce qu'**il comprend les étapes automatiques suivantes :
- une première étape de positionnement du dispositif selon l'une des revendications 1 à 4, sur le système automatique selon l'une quelconque des revendications 5 à 8 et de positionnement des au moins deux prolongateurs sur les au moins deux pompes, et de raccordement de ces prolongateurs (P1) aux flacons (16) contenant chacun un médicament radiopharmaceutique, ces flacons étant préalablement positionnés dans les moyens de confinement des rayonnements ionisants,
- une seconde étape de positionnement de la seringue patient (S1) munie de son protège-seringue blindé ainsi que du second connecteur de sécurité sur l'axe vertical en position la plus haute,
- une troisième étape de sélection des paramètres dans un logiciel de mise en seringue et de lancement du logiciel,
- une quatrième étape de positionnement automatique de la console de sélection par le biais de la commande linéaire horizontale, de positionnement automatique de la seringue patient en position basse face à l'appareil de mesure et de connexion d'un premier et d'un second connecteurs de sécurité par le biais de la commande verticale automatisée,
- une cinquième étape de remplissage partiel par le biais d'au moins un prolongateur d'au moins une seringue patient en fonction du médicament radiopharmaceutique et de l'activité radioactive souhaités,
- une sixième étape de déconnexion des connecteurs de sécurité (C1, C2) et de remontée de la seringue patient le long de l'axe linéaire vertical par le biais de la commande automatisée.

10. Procédé selon la revendication 9 dans lequel, dans l'étape de déconnexion des deux connecteurs (C1, C2), la commande automatisée (14) de l'axe linéaire vertical engendre la fermeture d'une fenêtre amovible au niveau du protège seringue.

11. Procédé selon la revendication 9, dans lequel, lorsqu'une dilution s'avère nécessaire, le second connecteur (C2) de la seringue patient (S1) est de nouveau connecté au premier connecteur (C1) du prolongateur (P1) correspondant au flacon (16), contenant la solution de dilution par la biais de la commande automatisée (14), après que la console de sélection (D1) ait effectué un un mouvement linéaire horizontal par le biais de la commande automatisée (15).

## Patentansprüche

1. Sterile Einweg-Vorrichtung zum Befüllen von Spritzen mit einem radiopharmazeutischen Medikament, die wenigstens zwei Verlängerer (P1) umfasst, welche jeweils an einem ersten Ende (E1) an eine Spinalnadel (A1) angeschlossen sind, die dazu ausgelegt ist, ein Fläschchen (16) aufzustoßen, das ein radiopharamazeutisches Medikament enthält und in Mitteln zum Einschließen ionisierender Strahlen positioniert ist, sowie an einem zweiten Ende (E2) an einen ersten Verbinder,
**dadurch gekennzeichnet, dass** dieser erste Verbinder ein Sicherheitsverbinder (C1) ist, der dazu ausgelegt ist, an einen zweiten Sicherheitsverbinder (C2) angeschlossen zu sein, der mit einer Patientenspritze (S1) verbunden ist, um das Befüllen dieser Patientenspritze zu ermöglichen, jegliche Kontamination durch Ausfließen zu vermeiden und beim Lösen ihrer Verbindung eine mikrobiologische Sicherheit zu gewährleisten, wobei der erste Sicherheitsverbinder so lange in einer geschlossenen Position ist, wie er nicht mit dem zweiten Sicherheitsverbinder verbunden ist, wobei die Verbindung dieses ersten und dieses zweiten Sicherheitsverbinders eine Fluidzirkulation ermöglicht,
- und dass die Vorrichtung ferner eine Konsole (D1) umfasst, an der wenigstens zwei erste Sicherheitsverbinder (C1) befestigt sind, und die auf einer horizontalen linearen Achse angeordnet ist, welche in einer horizontalen Translationsbewegung angetrieben werden kann.

2. Vorrichtung nach Anspruch 1, bei der das radiopharmazeutische Medikament mit Emittern von einfachen Photonen markiert ist.

3. Vorrichtung nach Anspruch 1, bei der das radiopharmazeutische Medikament mit Positronenemittern markiert ist.

4. Vorrichtung nach Anspruch 1, bei der der erste und der zweite Sicherheitsverbinder (C1, C2) dazu ausgelegt sind, in Reihe montiert zu sein.

5. Automatisches System zum Befüllen von Spritzen mit wenigstens einem radiopharmazeutischen Medikament, das in einem Fläschchen abgepackt ist, unter Verwendung einer Vorrichtung nach einem der vorhergehenden Ansprüche, umfassend:
- wenigstens zwei Pumpen (13), die dazu ausgelegt sind, jeweils wenigstens einen Verlängerer (P1) aufzunehmen,
- Mittel zum Einschließen von ionisierenden Strahlen von wenigstens zwei Fläschchen, die jeweils ein radiopharmazeutisches Medikament enthalten,
- wenigstens ein Gerät (10) zum Messen der Aktivität einer Patientenspritze (S1), die in einem Spritzenschutz eingeschlossen ist, wodurch die radionukleidische Identifikation des Radioelements oder die Identifikation der radionukleidischen Reinheit eines radiopharmazeutischen Medikaments ermöglicht wird, wobei dieses Messgerät (10) in einem abgeschirmten Schutzelement eingeschlossen ist,
- einen Absorber (12), der zwischen der Patientenspritze und dem Messgerät (10) positioniert ist und es ermöglicht, die Antwort des Messgeräts zu homogenisieren und gleichmäßig zu machen,
- eine vertikale lineare Achse (27), die mit automatischen Steuermitteln ausgestattet ist, und dazu ausgelegt ist, die mit ihrem Spritzenschutz und einem zweiten Sicherheitsverbinder (C2) ausgestattete Patientenspritze aufzunehmen,
- eine horizontale lineare Achse (28), die mit automatisierten Steuermitteln ausgestattet ist, die dazu ausgelegt sind, die Auswahlkonsole (D1) aufzunehmen,
- ein Informatiksystem (11), in welchem eine Spritzenbefüllungssoftware installiert ist, welche dazu ausgelegt ist, diese unterschiedlichen Steuermittel zu kontrollieren.

6. System nach Anspruch 5, bei dem die Mittel zum Einschließen (26) der ionisierenden Strahlen Schutzelemente aus Wolfram oder aus Blei sind, die auf einem Träger (17) befestigt sind.

7. System nach Anspruch 6, bei dem im Fall eines radiopharmazeutischen Medikaments, das mit Emittern von einfachen Photonen markiert ist, der Träger (17) auch das Heizen und/oder das Schütteln der Fläschchen gewährleistet.

8. System nach Anspruch 5, umfassend wenigstens einen Absorber (12), der zwischen der Patientenspritze (S1) und dem Messgerät (10) positioniert ist.

9. Verfahren zum Befüllen von Spritzen mit wenigstens einem radiopharmazeutischen Medikament, das in einem Fläschchen abgepackt ist, **dadurch gekennzeichnet, dass** es die folgenden automatischen Schritte umfasst:
- einen ersten Schritt der Positionierung der Vorrichtung nach einem der Ansprüche 1 bis 4 auf dem automatischen System nach einem der Ansprüche 5 bis 8 und der Positionierung der wenigstens zwei Verlängerer an den wenigstens zwei Pumpen sowie die Anschließens dieser Verlängerer (P1) an die Fläschchen (16), die jeweils ein radiopharmazeutisches Medikament enthalten, wobei diese Fläschchen zuvor in Mitteln zum Einschließen der ionisierenden Strahlen positioniert sind,
- einen zweiten Schritt der Positionierung der Patientenspritze (S1), die mit ihrem abgeschirmten Spritzenschutz sowie mit dem zweiten Sicherheitsverbinder ausgestattet ist, an der vertikalen Achse an der höchsten Position,
- einen dritten Schritt der Auswahl von Parametern in einer Spritzenbefüllungssoftware und des Startens der Software,
- einen vierten Schritt der automatischen Positionierung der Auswahlkonsole mittels der horizontalen linearen Steuerung, der automatischen Positionierung der Patientenspritze in einer niedrigen Position gegenüber dem Messgerät und des Verbindens eines ersten und eines zweiten Sicherheitsverbinders mittels der automatisierten vertikalen Steuerung,
- einen fünften Schritt des partiellen Befüllens wenigstens einer Patientenspritze mittels wenigstens eines Verlängerers als Funktion des gewünschten radiopharmazeutischen Medikaments und der gewünschten radioaktiven Aktivität,
- einen sechsten Schritt des Lösens der Verbindung der Sicherheitsverbinder (C1, C2) und des Wiederanhebens der Patientenspritze entlang der vertikalen linearen Achse mittels der automatisierten Steuerung.

10. Verfahren nach Anspruch 9, bei dem im Schritt des Lösens der Verbindung der zwei Verbinder (C1, C2) die automatisierte Steuerung (14) der vertikalen linearen Achse das Schließen eines abnehmbaren Fensters im Bereich des Spritzenschutzes bewirkt.

11. Verfahren nach Anspruch 9, bei dem dann, wenn sich eine Verdünnung als erforderlich herausstellt, der zweite Verbinder (C2) der Patientenspritze (S1) erneut mit dem ersten Verbinder (C1) des dem Fläschchen (16) entsprechenden Verlängerers (P1) verbunden wird, in dem die Verdünnungslösung erhalten ist, und zwar mittels der automatisierten Steuerung (14), nachdem die Auswahlkonsole (D1) mittels der automatisierten Steuerung (15) eine horizontale lineare Bewegung ausgeführt hat.

## Claims

1. A single-use sterile device for filling syringes with a radiopharmaceutical drug, comprising at least two extensions (P1), each connected, at a first end (E1), to a spiral needle (A1) suitable for percuss a bottle (16), containing a radiopharmaceutical drug, positioned in ionising radiation confinement means and, at a second end (E2), to a first connector,
**characterised in that** this first connector is a safety connector (C1) suitable for being connected to a second safety connector (C2) rigidly connected to a patient syringe (S1) to enable filling of this patient syringe, prevent any contamination due to flow and ensure microbiological safety during the disconnection thereof, the first safety connector being in a closed position while it is not connected to the second safety connector, the connection of this first and this second safety connectors enabling fluid circulation,
and **in that** the device further comprises a console (D1) whereon at least two first safety connectors (C1) are attached and which is arranged on a horizontal linear axis suitable for being moved with a horizontal translational movement.

2. The device according to claim 1, wherein the radiopharmaceutical drug is labelled with single-photon emitters.

3. The device according to claim 1, wherein the radiopharmaceutical drug is labelled with positon emitters.

4. The device according to claim 1, wherein the first and second safety connectors (C1, C2) are suitable for being assembled in series.

5. An automatic system for filling syringes with at least one radiopharmaceutical drug, packaged in bottles, using a device according to any of the above claims, comprising:
- at least two pumps (13), for each suitable receiving at least one extension (P1),
- ionising radiation confinement means, of at least two bottles each containing a radiopharmaceutical drug,
- at least one apparatus (10) for measuring the activity of a patient syringe (S1) confined in a syringe shield enabling the radionuclide identification of the radioelement or the identification of the radionuclide purity of a radiopharmaceutical drug, this measurement apparatus (10) being confined in an enclosed protection,
- an absorber (12) positioned between the patient syringe and the measurement apparatus (10) for homogenising and standardising the response of the measurement apparatus,
- a vertical linear axis (27) provided with automated control means, suitable for receiving a patient syringe equipped with the syringe shield thereof and a second safety connector (C2),
- a horizontal linear axis (28) equipped with automated control means suitable for receiving the selection console (D1),
- a computer system (11), wherein a syringe filling software is installed, suitable for controlling these various control means.

6. The system according to claim 5, wherein the ionising radiation confinement means (26) are tungsten or lead protections mounted on a supporting member (17).

7. The system according to claim 6, wherein, in the case of a radiopharmaceutical drug labelled with single-photon emitters, the supporting member (17) also carries out the heating and/or stirring of the bottles.

8. The system according to claim 5, comprising at least one absorber (12) positioned between the patient syringe (S1) and the measurement apparatus (10).

9. A method for filling syringes with at least one radiopharmaceutical drug packaged in bottles, **characterised in that** it comprises the following automatic steps:
- a first step for positioning the device according to any of claims 1 to 4, on the automatic system according to any of claims 5 to 8 and for positioning the at least two extensions on the at least two pumps, and for connecting these extensions (P1) to the bottles (16) each containing a radiopharmaceutical drug, these bottles being previously positioned in the ionising radiation confinement means,
- a second step for positioning the patient syringe (S1) equipped with the enclosed syringe shield along with the second safety connector on the vertical axis in the uppermost position,
- a third step for selecting the parameters in syringe filling software and running the software,
- a fourth step for automatically positioning the selection console via the horizontal linear control, automatically positioning the patient syringe in the low position facing the measurement apparatus and connecting a first and a second safety connectors via the automated vertical control,
- a fifth step for partial filling via at least one extension of at least one patient syringe according to the sought radiopharmaceutical drug and radioactive activity,
- a sixth step for disconnecting the safety connectors (C1, C2) and lifting the patient syringe along the vertical linear axis via the automated control.

10. The method according to claim 9 wherein, in the step for disconnecting the two connectors (C1, C2), the automated control (14) of the vertical linear axis generates the closure of a removable window on the syringe shield.

11. The method according to claim 9, wherein, when a dilution is proved to be necessary, the second connector (C2) of the patient syringe (S1) is once again connected to the first connector (C1) of the extension (P1) corresponding to the bottle (16), containing the dilution solution via the automated control (14), after the selection console (D1) has performed a horizontal linear movement via the automated control (15).
